# EUROPÄISCHE PATENTSCHRIFT

(11) **EP 1 767 514 B1**
(45) Veröffentlichungstag und Bekanntmachung des Hinweises auf die Patenterteilung: **31.03.2010**
(21) Anmeldenummer: 06120341.0
(22) Anmeldetag: 08.09.2006
(51) Int. Cl.: C07C 43/215, C07C 41/32

(54) **Verfahren zur Isomerisierung von Alkenylalkoxybenzolen**
Process for the isomerisation of alkenylalkoxybenzenen
Procédé d' isomérisation des benzènes alkoxyalcénylés

(30) Priorität: 26.09.2005 DE 102005045945
(43) Veröffentlichungstag der Anmeldung: 28.03.2007
(73) Patentinhaber: Symrise GmbH & Co. KG, 37603 Holzminden (DE)
(72) Erfinder: Kuhn, Walter, 37603, Holzminden (DE); Funk, Hans-Ulrich, 37697, Lauenförde (DE); Senft, Gerhard, 37603, Holzminden (DE); Dittert, Christoph, 37671, Höxter (DE); Stephan, Torsten, 37603, Holzminden (DE)
(74) Vertreter: Eisenführ, Speiser & Partner

(56) Entgegenhaltungen:
- GB-A- 317 347
- DATABASE CAPLUS [Online] CHEMICAL ABSTRACTS SERVICE, COLUMBUS, OHIO, US; LE, NGOC THACH ET AL: "Isomerization of methyl chavicol" XP002417152 gefunden im STN Database accession no. 1998:417121 & TAP CHI HOA HOC , 35(4), 23-26, 30 CODEN: TCHHDC; ISSN: 0378-2336, 1997,
- SUZUKI, SHOHEI ET AL: "Application of electrogenerated triphenylmethyl anion as a base for alkylation of arylacetic esters and arylacetonitriles and isomerization of allylbenzenes" CANADIAN JOURNAL OF CHEMISTRY , 72(2), 357-61, Bd. 72, Nr. 2, 1994, Seiten 357-361, XP009077753

## Beschreibung

Die vorliegende Erfindung betrifft ein Verfahren zur katalytischen Doppelbindungsisomerisierung (im Folgenden: Isomerisierung) von Alkenylalkoxybenzolen [alkoxysubstituierte (2-Alkenyl)-benzole] (mit einer nichtkonjugierten Doppelbindung in der Alkenylgruppe) der Formel **A**

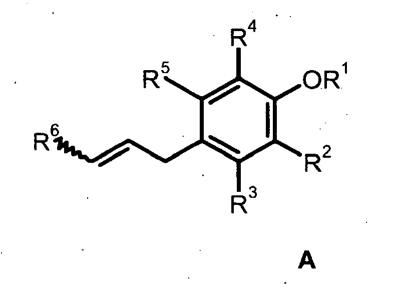

in Gegenwart einer katalytisch wirksamen Menge an Alkali- und/oder Erdalkali-C₁-C₆-alkoholaten zu den entsprechenden Alkenylalkoxybenzolen [alkoxysubstituierten (1-Alkenyl)-benzole] (mit einer Doppelbindung in der Alkenylgruppe, die mit dem Benzolring konjugiert ist) der Formel **B**.

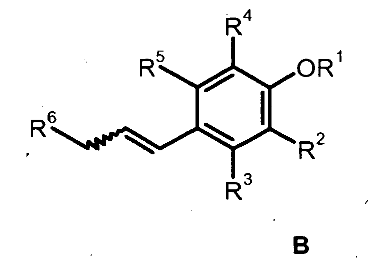

Die geschlängelte Linie in den Formeln **A** und **B** bedeutet, dass die Doppelbindung cis- oder trans-konfiguriert sein kann; zur Bedeutung der Reste R¹ bis R⁶ siehe unten.

Verbindungen der Formel **B** weisen ein größeres konjugiertes System als Verbindungen der Formel **A** auf und sind somit grundsätzlich stabiler. Des weiteren weisen Verbindungen der Formel **B** üblicherweise interessante Geruchsnoten auf. Somit besteht seit langem das Bedürfnis Alkenylalkoxybenzole der Formel **B** zu synthetisieren.

In DE 1 936 727 wird die Isomerisierung von alkenylaromatischen Verbindungen mittels Ruthenium bzw. Osmium-Katalysatoren beschrieben. Gemäß Helvetica Chimica Acta 1992, 75(5), 1604 werden Hexaaquaruthenium-Katalysatoren eingesetzt.

In DE 2 157 534 wird als Katalysator für eine solche Isomerisierung ein Gemisch aus metallischem Natrium, Natriumhydroxid und Aluminiumoxid vorgeschlagen.

In GB 525705 wird die Doppelbindungsisomerisierung von alkenylaromatischen Verbindungen unter Einsatz von Alkalimetallhydroxiden und Glykolen oder Polyglykolen als Verdünnungsmittel für die Alkalimetallhydroxide beschrieben. Unter anderem wird Eugenolmethylether in Gegenwart von Kaliumhydroxid, Diethylenglykolmonoethylether und Ethanolamin bei 160-180°C umgesetzt. Es wurde dabei Isoeugenolmethylether mit 95%iger Ausbeute erhalten, wobei das trans-Doppelbindungsisomere überwog.

In Zhurnal Organicheskoi Khimii 1988, 24(6), 1248-1253 wurde die Kinetik der Isomerisierung von Allylbenzol (keine Verbindung der Formel **A**) in Gegenwart von Lithium-tert-butanolat in N,N-Dimethylacetamid bei 25°C bzw. in Gegenwart von Kalium-tert-butanolat in tert.-Butanol bei 75°C untersucht. Unter Verwendung einer 0,5 molaren Lösung des aktiveren Katalysatorsystems Lithium-tert-butanolat in N,N-Dimethylacetamid wurde die Isomerisierungsrate anscheinend bis zu einer etwa 4 molaren Konzentration an Allylbenzol gemessen. Es wurde festgestellt, dass mit steigender Konzentration des Allylbenzols die Isomerisierungsrate kleiner, d.h. die Reaktion langsamer, wurde. Unter Verwendung einer 0,5 molaren Lösung von Kalium-tert-butanolat in tert.-Butanol wurde die Isomerisierungsrate anscheinend bis zu einer etwa 1 molaren Konzentration an Allylbenzol gemessen. Ferner wurde die Isomerisierungsgeschwindigkeit in Abhängigkeit von der Katalysatorkonzentration bei einer 0,1 molaren Konzentration von Allylbenzol untersucht. Hierbei lag die niedrigste untersuchte molare Katalysatormenge an Lithium-tert-butanolat in N,N-Dimethylacetamid anscheinend bei etwa 31 mol%, im Falle von Kalium-tert-butanolat in tert.-Butanol bei über 100 mol%, jeweils bezogen auf Allylbenzol. Es wurden auch die Hammett-Konstanten einiger kernsubstituierter Allylbenzole ermittelt, in diesem Rahmen wurde unter anderem die Geschwindigkeitskonstante der Isomerisierung von Methylchavicol (p-Methoxy-allylbenzol; Estragol) bestimmt. Über die jeweiligen Ausbeuten und die erhaltenen Doppelbindungskonfigurationen wird insgesamt nicht berichtet.

In dem vietnamesischen Dokument Tap Chi Hoa Hoc 1997, 35(4), 23-26 (Chemical Abstracts 129:135940) werden Ergebnisse zur Isomerisierung von Methylchavicol zu Anethol (1-Methoxy-4-(1-propenyl)benzol) in Gegenwart von KOH, Kalium-tert.-butanolat bzw. KF/Al₂O₃ präsentiert. Es wird beschrieben, dass die lösungsmittelfreie Durchführung der Isomerisierung zu einer höheren Reaktionsgeschwindigkeit führt, ebenso wie der Zusatz von 5% eines Phasentransfer-Katalysators. Ferner wurde dort festgestellt, dass die Bestrahlung mit Mikrowellen die Reaktionszeit drastisch verkürzt. Die Ausbeute wurde dabei allerdings in keinem einzigen Fall gesteigert. Ein 1 : 1 Molverhältnis von Kalium-tert.-butanolat zu Methylchavicol wird dort als optimal angesehen.

Die besten Isomerisierungsergebnisse wurden bei 80°C bzw. 110°C unter Verwendung einer äquimolaren Menge an Kalium-tert.-butanolat erhalten, wobei nach 1 bis 1,5 Stunden Reaktionszeit die Ausbeute an Anethol bei 93% lag. Zu der erhaltenen Doppelbindungsgeometrie des Anethols werden keine Aussagen gemacht.

Anhand wissenschaftlicher Analysen ist festgestellt worden, dass cis-Isomere von Verbindungen der Formel **B** häufig toxischer als die trans-Isomere dieser Verbindungen sind. Insbesondere ist cis-Anethol 10 bis 20 mal toxischer als das trans-Anethol (vgl. R. Hänsel, J. Hölzel, "Lehrbuch der pharmazeutischen Biologie", 1996, S. 162 ff.) und für cis-Asaron (β-Asaron, 1,2,4-Trimethoxy-5-(Z)-1-propenylbenzol) ist im Gegensatz zu trans-Asaron (α-Asaron) eine kanzerogene Eigenschaft im Tierversuch festgestellt worden (http://www.omikron-online.de/cyberchem/aroinfo/asaron a.htm). Weiterhin entfalten im Allgemeinen trans-Isomere der Verbindung der Formel **B** eine interessantere geruchliche Note. Somit besteht ein Bedarf an einem Herstellungsverfahren für Verbindungen der Formel **B** mit hoher Ausbeute an dem jeweiligen trans-Isomer.

Der vorliegenden Erfindung lag entsprechend die Aufgabe zugrunde, ein verbessertes Verfahren zur Isomerisierung von Verbindungen der Formel **A** zu den Verbindungen der Formel **B** zu entwickeln, so dass die gewünschten Verbindungen der Formel **B** mit möglichst hoher Ausbeute und mit einem möglichst hohen Anteil der (geruchlich wertvolleren) trans-Doppelbindungsisomere erhalten wird. Ferner sollte das Verfahren wirtschaftlich und im technischen Maßstab durchführbar sein, sowie die Verbindungen der Formel **B** in hoher Reinheit (und guter geruchlicher) Qualität liefern.

Die gestellte Aufgabe wird erfindungsgemäß gelöst durch ein Verfahren zur Herstellung einer Verbindung der Formel **B** aus einer Verbindung der Formel **A** 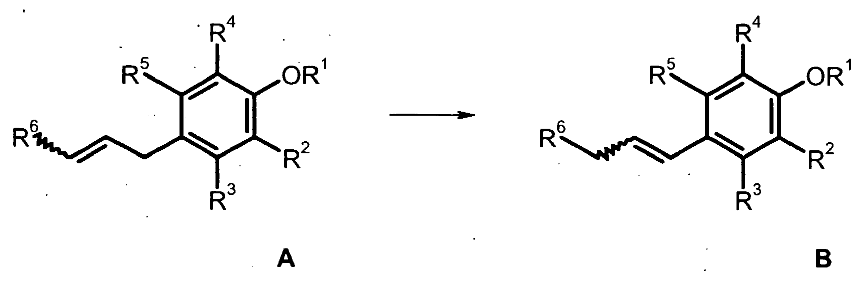 wobei jeweils gilt:
R¹ bedeutet einen Alkylrest mit 1 bis 6 C-Atomen,
R², R³, R⁴, R⁵ bedeuten jeweils unabhängig voneinander Wasserstoff oder einen (verzweigten oder unverzweigten) Alkyl- oder Alkoxyrest mit 1 bis 6 C-Atomen und
R⁶ bedeutet Wasserstoff oder einen (verzweigten oder unverzweigten) Alkylrest mit 1 bis 6 C-Atomen;
   oder
R¹ und R² sind miteinander verknüpft, so dass ein insgesamt 5 bis 7-gliedriger Ring gebildet ist, der insgesamt 3 bis 6 C-Atome und 1 oder 2 Sauerstoffatome umfasst (vgl. die Beispiele unten), und
R³, R⁴, R⁵ bedeuten jeweils unabhängig voneinander Wasserstoff oder einen (verzweigten oder unverzweigten) Alkyl- oder Alkoxyrest mit 1 bis 6 C-Atomen, und
R⁶ bedeutet Wasserstoff oder einen (verzweigten oder unverzweigten) Alkylrest mit 1 bis 6 C-Atomen,
in Gegenwart von 0,1 bis 10 mol%, eines Alkali- oder Erdalkalialkoholats mit 1 bis 6 C-Atomen, bezogen auf die eingesetzte Menge an Verbindung der Formel **A**.

Bevorzugte Alkylreste mit 1-6 C-Atomen sind: Methyl, Ethyl, n-Propyl, Isopropyl, Butyl, Isobutyl, sec.- Butyl, tert.-Butyl, n-Pentyl, Isopentyl, tert.-pentyl, n-Hexyl, iso-Hexyl und tert.-Hexyl.

Bevorzugte Alkoxyreste mit 1-6 C-Atomen sind: Methoxy, Ethoxy, Propoxy, Isopropoxy, Butoxy, Isobutoxy, tert.-butoxy, Pentoxy, Isopentoxy, Hexoxy und Isohexoxy.

Besonders überraschend ist, dass das erfindungsgemäße Verfahren zur Isomerisierung, das mit sehr geringen Katalysatormengen auskommt, mit hervorragender Ausbeute Verbindungen der Formel **B** liefert und mit sehr hoher Selektivität zu den entsprechenden trans-Doppelbindungsisomeren der Formel **B** führt.

Als Katalysatoren werden in den erfindungsgemäßen Verfahren Alkali- und/oder Erdalkalimetallalkoholate mit 1 bis 6 Kohlenstoffatomen eingesetzt. Vorzugsweise werden neben diesen Alkali- und Erdalkalimetallalkoholaten keine weiteren, d.h. keine zusätzlichen, für eine Doppelbindungsisomerisierung aktiven Katalysatoren eingesetzt.

Bevorzugte Katalysatoren sind Alkalialkoholate, vorzugsweise Natrium- und Kaliumalkoholate, mit 1 bis 6 Kohlenstoffatomen, vorzugsweise mit 1 bis 4 Kohlenstoffatomen. Daneben sind auch bestimmte Erdalkalialkoholate bevorzugt.

Als vorzugsweise in erfindungsgemäßen Verfahren einzusetzende Alkali- bzw. Erdalkalimetallalkoholate seien genannt: Kalium-tert-butanolat, Natrium-tert-butanolat, Lithium-tert-butanolat, Natriumethanolat, Kaliumethanolat, Lithiumethanolat, Natriummethanolat, Kaliummethanolat, Lithiummethanolat, Natriumisopropanolat, Kaliumisopropanolat, Lithiumisopropanolat, Magnesiumethanolat, Magnesiummethanolat, Calciumethanolat und Calciummethanolat. Ferner bevorzugt sind deren Mischungen.

Insbesondere bevorzugt sind Natriummethanolat, Natriumethanolat, Natriumisopropanolat, Natrium-tert-butanolat, Kaliummethanolat, Kaliumethanolat, Kalium-isopropanolat und Kalium-tert-butanolat.

Besonders gute Ergebnisse werden mit den Katalysatoren Kalium-tert-butanolat und Natrium-tert-butanolat (tertiären Alkoholaten) erhalten. Insbesondere mit Kalium-tert-butanolat werden eine ausgezeichnete Ausbeute, eine hohe (geruchliche) Reinheit sowie eine sehr hohe Selektivität zu den trans-Doppelbindungsisomeren der Formel **B** erzielt.

In einer erfindungsgemäß bevorzugten Ausführungsform werden die Verfahrensbedingungen so eingestellt, dass durch die erfindungsgemäße Isomerisierung ein Verhältnis der trans- : cis- Doppelbindungsisomere der Formel **B** im Bereich von 80 : 20 bis 99 : 1, vorzugsweise im Bereich von 90 : 10 bis 98 : 2 erreicht wird.

Die erfindungsgemäße Isomerisierung erfolgt in Gegenwart von 0,1 bis 10 mol% (entsprechend 0,001 bis 0,1 molare Äquivalente) der besagten Katalysatoralkoholate, bevorzugt sind 0,5 bis 8 mol%, besonders bevorzugt 1 bis 5 mol%. Die molaren Äquivalente beziehen sich hierbei auf die eingesetzte Menge an zu isomerisierender Verbindung der Formel **A**.

Bevorzugte Verbindungen der Formel **A** bzw. der Formel **B** sind solche, in denen gilt:
R¹ bedeutet einen Alkylrest mit 1 bis 6 C-Atomen,
R², R³, R⁴, R⁵ bedeuten jeweils unabhängig voneinander Wasserstoff oder Methoxy, mit der Maßgabe, dass zumindest einer der Reste R², R³, R⁴, R⁵ Methoxy ist und
R⁶ bedeutet Wasserstoff oder Methyl;
   oder
R¹ und R² sind miteinander verknüpft, so dass ein insgesamt 5 oder 6-gliedriger Ring gebildet ist, der insgesamt 3 oder 4 C-Atome und 1 oder 2 Sauerstoffatome umfasst, und
R³, R⁴, R⁵ bedeuten jeweils unabhängig voneinander Wasserstoff oder Methoxy und
R⁶ bedeutet Wasserstoff oder Methyl.

Hier seien beispielsweise die Isomerisierung von Methylchavicol zu Anethol und von 2,4-Dimethoxy-1-allylbenzol zu 2,4-Dimethoxy-1-propenylbenzol genannt.

Besonders bevorzugte Verbindungen der Formel **A** sind solche, in denen R⁶ Wasserstoff bedeutet und R² die Bedeutung OR² gemäß der nachfolgenden Formel **A1** besitzt, 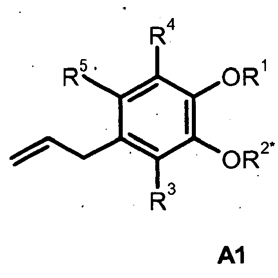 in der gilt:
R¹ bedeutet einen Alkylrest mit 1 bis 6 C-Atomen;
R^{2*} bedeutet Methyl;
R³, R⁴ und R⁵ bedeuten jeweils unabhängig voneinander Wasserstoff oder Methoxy, mit der Maßgabe, dass zumindest ein Rest R³, R⁴ oder R⁵ Wasserstoff bedeutet,
   oder
R¹ und R²* sind miteinander verknüpft, so dass ein insgesamt 5 oder 6-gliedriger Ring gebildet ist, der insgesamt 3 oder 4 C-Atome und 2 Sauerstoffatome umfasst;
R³, R⁴ und R⁵ bedeuten jeweils unabhängig voneinander Wasserstoff oder Methoxy, mit der Maßgabe, dass zumindest ein Rest R³, R⁴ oder R⁵ Wasserstoff bedeutet.

Ganz besonders bevorzugt in den erfindungsgemäßen Verfahren (Isomerisierungen) einzusetzende Verbindungen der Formel **A** sind solche der Formeln **A2** und **A3**: 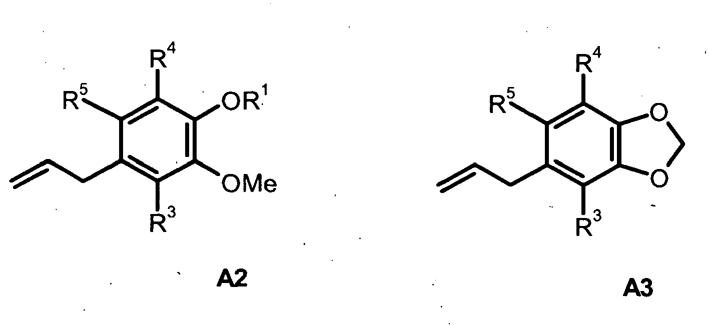 wobei für **A2** und **A3** gilt:
R³, R⁴ und R⁵ bedeuten jeweils unabhängig voneinander Wasserstoff oder Methoxy, mit der Maßgabe, dass zumindest ein Rest R³, R⁴ oder R⁵ Wasserstoff bedeutet,
und zusätzlich in Formel **A2** gilt:
R¹ bedeutet einen Alkylrest mit 1 bis 6 C-Atomen.

Eine Verbindung der Formel **A3** ist eine Verbindung der Formel **A**, in der R¹ und R² miteinander verknüpft sind, so dass ein insgesamt 5-gliedriger Ring gebildet ist, der insgesamt 3 C-Atome und 2 Sauerstoffatome umfasst.

Ganz besonders bevorzugt in den erfindungsgemäßen Verfahren (Isomerisierungen) einzusetzende Verbindungen der Formel **A2** sind: Methyleugenol (Eugenolmethylether, R¹ = Methyl, R³, R⁴ und R⁵ = H), Ethyleugenol (R¹ = Ethyl, R³, R⁴ und R⁵ = H), iso-Amyleugenol (R¹ = iso-Amyl, R³, R⁴ und R⁵ = H), und Elemicin (R¹ = Methyl, R⁴ = Methoxy, R³ und R⁵ = H).

Ganz besonders bevorzugt in den erfindungsgemäßen Verfahren einzusetzende Verbindungen der Formel **A3** sind: Safrol (R³, R⁴ und R⁵ = H), Myristicin (R⁴ = Methoxy, R³ und R⁵ = H), Apiol (R³ und R⁴ = Methoxy, R⁵ = H) und Dillapiol (R⁴ und R⁵ = Methoxy, R³= H).

Ganz besonders bevorzugte nach der erfindungsgemäßen Isomerisierung Verbindungen der Formel **B** sind entsprechend: Methylisoeugenol (Isoeugenolmethylether), Ethylisoeugenol, iso-Amylisoeugenol, Isoelemicin, Isosafrol, Isomyristicin, Isoapiol, Dillisoapiol.

Die Reaktionstemperatur bei Durchführung der erfindungsgemäßen Verfahren (Isomerisierungen) liegt vorzugsweise im Bereich von 40 bis 190°C, bevorzugt im Bereich von 60 bis 160°C. Sofern als Katalysatoren die bevorzugten tertiären Alkalialkoholate, wie beispielsweise Kalium-tert-butanolat, verwendet werden, sind Temperaturen im Bereich von 60 bis 100°C bevorzugt.

Die Isomerisierung kann erfindungsgemäß in einem inerten Verdünnungsmittel durchgeführt werden, vorzugsweise wird die Isomerisierung verdünnungsmittelfrei durchgeführt.

Wird die erfindungsgemäße Isomerisierung in einem inerten Verdünnungsmittel durchgeführt, so sind (gesättigte) Kohlenwasserstoffe, vorzugsweise mit 10 bis 16 Kohlenstoffatomen, wie beispielsweise Dodecan, oder Ether, insbesondere solche mit einem Siedepunkt oberhalb der Reaktionstemperatur, vorzugsweise Diphenylether, bevorzugt. Ferner kann auch das in der Isomerisierung zu bildende Produkt der Formel **B** als Verdünnungsmittel eingesetzt werden. Vorzugsweise wird kein Glykol oder Polyglykol als Verdünnungsmittel eingesetzt. Ferner wird vorzugsweise kein N,N-Dimethylacetamid, kein tert.-Butanol und kein DMSO als Verdünnungsmittel eingesetzt.

Die Isomerisierung wird erfindungsgemäß vorzugsweise in Abwesenheit eines Phasentransfer-Katalysators durchgeführt, ebenso in Abwesenheit einer Mikrowellen-Bestrahlung.

Die Isomerisierung kann erfindungsgemäß kontinuierlich oder diskontinuierlich durchgeführt werden. Die eingesetzten Verbindungen der Formel **A** können entweder von Anfang an zusammen mit dem Katalysator in das Reaktionsgefäß gefüllt werden oder je nach Reaktionsverlauf kontinuierlich oder schubweise in das Reaktionsgefäß eingebracht werden.

Der Reaktionsverlauf bzw. das Fortschreiten der erfindungsgemäßen Isomerisierung kann beispielsweise mittels Gaschromatographie verfolgt werden. Da die Reaktion in hoher trans-Selektivität verläuft, fallen die Endprodukte nach Herauswaschen des Katalysators in oftmals ausreichend hoher Reinheit an. Eine weitere Aufreinigung kann beispielsweise durch Destillation erfolgen.

Die folgenden Beispiele erläutern die Erfindung. Sofern nicht anders angegeben beziehen sich alle Angaben auf das Gewicht.

### Beispiel 1: Isomerisierung von Eugenolmethylether (Methyleugenol) zu Isoeugenolmethylether (Methylisoeugenol) mittels Natriummethanolat

178 g Eugenolmethylether (1 mol) und 1,1 g Natriummethanolat (0,02 mol) wurden bei 130°C über einen Zeitraum von 4 Stunden gerührt. Anschließend wurde mit 4 g 10%iger NaCl-Lösung gewaschen. Die organische Phase (171 g) bestand im Wesentlichen aus 81,1 % trans-Isoeugenolmethylether, 18,1% cis-Isoeugenolmethylether und 0,6% Eugenolmethylether. Das Produkt destillierte bei 123°C und 10 mbar Vakuum über Kopf. Es verblieben 12 g Rückstand.

### Beispiel 2: Isomerisierung von Eugenolmethylether (Methyleugenol) zu Isoeugenolmethylether (Methylisoeugenol) mittels Kalium-tert.-butanolat

178 g Eugenolmethylether (1 mol) und 2 g Kaliumtertiärbutanolat (0.018 mol) wurden bei 70-75°C über einen Zeitraum von 6 Stunden gerührt. Anschließend wurde mit 20 g 15%iger NaCl-Lösung gewaschen. Die organische Phase (175 g) bestand im Wesentlichen aus 95,7% trans-Isoeugenolmethylether, 3,9% cis-Isoeugenolmethylether und 0,1% Eugenolmethylether. Das Produkt destillierte bei 123°C und 10 mbar Vakuum über Kopf und wies eine gute geruchliche Qualität auf. Es verblieben 10 g Rückstand.

### Beispiel 3: Isomerisierung von Eugenolmethylether zu Isoeugenolmethylether mittels KOH (nicht erfindungsgemäß)

178 g Eugenolmethylether (1 mol) und 3,6 g Kaliumhydroxid (0.064 mol) wurden bei 120°C über einen Zeitraum von 9 Stunden gerührt. Anschließend wurde mit 20 g 15%iger NaCl-Lösung gewaschen. Die organische Phase bestand im Wesentlichen aus 71,9% trans-Isoeugenolmethylether, 16,7% cis-Isoeugenolmethylether und 11,1% Eugenolmethylether.

## Patentansprüche

1. Verfahren zur Herstellung einer Verbindung der Formel **B** aus einer Verbindung der Formel **A** 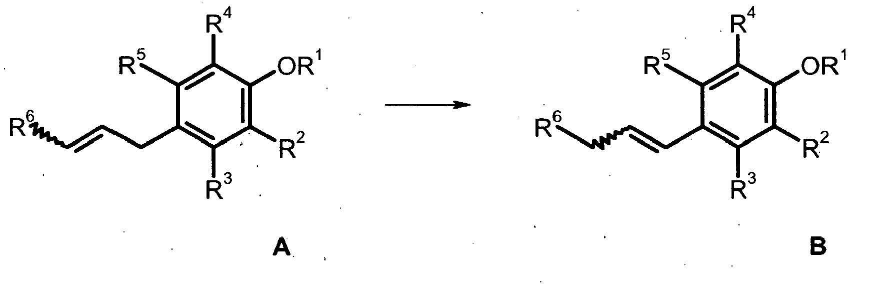 wobei jeweils gilt:
R¹ bedeutet einen Alkylrest mit 1 bis 6 C-Atomen,
R², R³, R⁴, R⁵ bedeuten jeweils unabhängig voneinander Wasserstoff oder einen Alkyl- oder Alkoxyrest mit 1 bis 6 C-Atomen und
R⁶ bedeutet Wasserstoff oder einen Alkylrest mit 1 bis 6 C-Atomen
oder
R¹ und R² sind miteinander verknüpft, so dass ein insgesamt 5 bis 7-gliedriger Ring gebildet ist, der insgesamt 3 bis 6 C-Atome und 1 oder 2 Sauerstoffatome umfasst, und
R³, R⁴, R⁵ bedeuten jeweils unabhängig voneinander Wasserstoff oder einen Alkyl- oder Alkoxyrest mit 1 bis 6 C-Atomen und
R⁶ bedeutet Wasserstoff oder einen Alkylrest mit 1 bis 6 C-Atomen
in Gegenwart von 0,1 bis 10 mol%, eines Alkali- oder Erdalkalialkoholats mit 1 bis 6 C-Atomen, bezogen auf die eingesetzte Menge an Verbindung der Formel **A.**

2. Verfahren nach Anspruch 1, wobei die Isomerisierung verdünnungsmittelfrei durchgeführt wird.

3. Verfahren nach einem der vorangehenden Ansprüche, wobei die Isomerisierung in Gegenwart von 0,5 bis 8 mol% des Alkali- oder Erdalkalialkoholats mit 1 bis 6 C-Atomen durchgeführt wird, bezogen auf die Menge der eingesetzten Verbindung der Formel **A.**

4. Verfahren nach einem der vorangehenden Ansprüche, wobei das Alkali- oder Erdalkalialkoholat ein Natrium- oder Kaliumalkoholat mit 1 bis 4 C-Atomen ist.

5. Verfahren nach einem der vorangehenden Ansprüche, wobei die Verbindung der Formel **A** eine Verbindung der Formel **A1** ist 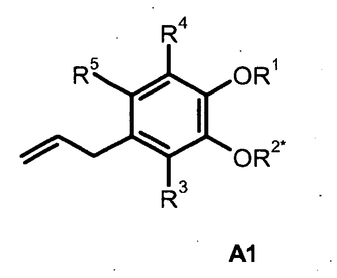 wobei gilt:
R¹ bedeutet einen Alkylrest mit 1 bis 6 C-Atomen;
R² bedeutet Methyl;
R³, R⁴ und R⁵ bedeuten jeweils unabhängig voneinander Wasserstoff oder Methoxy, mit der Maßgabe, dass zumindest ein Rest R³, R⁴ oder R⁵ Wasserstoff bedeutet,
oder
R¹ und R^{2*} sind miteinander verknüpft, so dass ein insgesamt 5 oder 6-gliedriger Ring gebildet ist, der insgesamt 3 oder 4 C-Atome und 2 Sauerstoffatome umfasst,
R³, R⁴ und R⁵ bedeuten jeweils unabhängig voneinander Wasserstoff oder Methoxy, mit der Maßgabe, dass zumindest ein Rest R³, R⁴ oder R⁵ Wasserstoff bedeutet.

6. Verfahren nach einem der vorangehenden Ansprüche, wobei die Verbindung der Formel **A** gewählt ist aus der Gruppe bestehend aus Methyleugenol, Ethyleugenol, iso-Amyleugenol, Elemicin, Safrol, Myristicin, Apiol und Dillapiol.

7. Verfahren nach einem der vorangehenden Ansprüche, wobei das trans- : cis-Verhältnis der Doppelbindungsisomere der Formel **B** im Bereich von 80 : 20 bis 99 : 1, vorzugsweise im Bereich von 90 : 10 bis 98 : 2, liegt.

## Claims

1. Process for the preparation of a compound of formula **B** from a compound of formula **A:** 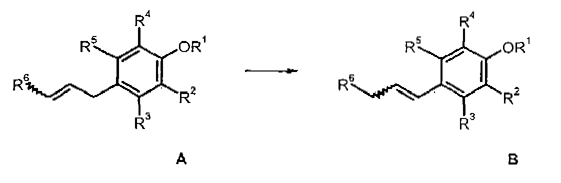 in which formulae;
R¹ is an alkyl radical having 1 to 6 C atoms,
R² R³, R⁴ and R⁵ independently of one another are each hydrogen or an alkyl or alkoxy radical having 1 to 6 C atoms, and
R⁶ is hydrogen or an alkyl radical having 1 to 6 C atoms,
or
R¹ and R² are coupled together to form a ring with a total of 5 to 7 members which comprises a total of 3 to 6 C atoms and 1 or 2 oxygen atoms,
R³, R⁴ and R⁵ independently of one another are each hydrogen or an alkyl or alkoxy radical having 1 to 6 C atoms, and
R⁶ is hydrogen or an alkyl radical having 1 to 6 C atoms,
in the presence of 0.1 to 10 mol% of an alkali metal or alkaline earth metal alcoholate having 1 to 6 C atoms, based on the amount of compound of formula **A** used.

2. Process according to Claim 1 wherein the isomerization is carried out without a diluent.

3. Process according to one of the preceding claims wherein the isomerization is carried out in the presence of 0.5 to 8 mol% of the alkali metal or alkaline earth metal alcoholate having 1 to 6 C atoms, based on the amount of compound of formula **A** used.

4. Process according to one of the preceding claims wherein the alkali metal or alkaline earth metal alcoholate is a sodium or potassium alcoholate having 1 to 4 C atoms.

5. Process according to one of the preceding claims wherein the compound of formula **A** is a compound of formula Al: 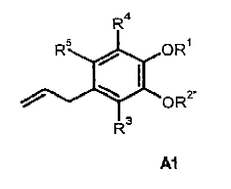 in which:
R¹ is an alkyl radical having 1 to 6 C atoms,
R² is methyl, and
R³, R⁴ and R⁵ independently of one another are each hydrogen or methoxy, with the proviso that at least one radical R³, R⁴ or P⁵ is hydrogen,
or
R¹ and R² are coupled together to form a ring with a total of 5 or 6 members which comprises a total of 3 or 4 C atoms and 2 oxygen atoms, and
R³, R⁴ and R⁵ independently of one another are each hydrogen or methoxy, with the proviso that at least one radical R³, R⁴ or R⁵ is hydrogen.

6. Process according to one of the preceding claims wherein the compound of formula **A** is selected from the group comprising methyleugenol, ethyleugenol, isoamyl-eugenol, elemicin, safrol, myristicin, apiol and dillapiol.

7. Process according to one of the preceding claims wherein the trans:cis ratio of the double bond isomers of formula **B** ranges from 80:20 to 99:1, preferably from 90:10 to 98:2.

## Revendications

1. Procédé pour la préparation d'un composé de la formule B à partir d'un composé de la formule A 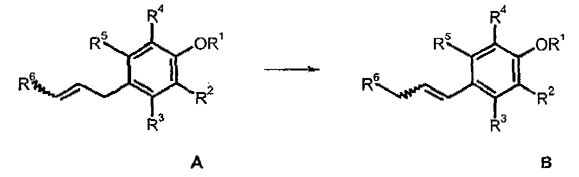 pour lequel on a :
R¹ signifie un radical alkyle avec 1 à 6 atomes de carbone,
R², R³, R⁴, R⁵ signifient respectivement, indépendamment l'un de l'autre, un hydrogène ou un radical alkyle ou alcoxyle avec 1 à 6 atomes de carbone et
R⁶ signifie un hydrogène ou un radical alkyle avec 1 à 6 atomes de carbone
ou
R¹ et R² sont reliés ensemble, de telle sorte qu'un cycle est formé, de 5 à 7 éléments en tout, qui comporte en tout 3 à 6 atomes de carbone et 1 ou 2 atomes d'oxygène, et
R³, R⁴, R⁵ signifient respectivement, indépendamment l'un de l'autre, un hydrogène ou un radical alkyle ou alcoxyle avec 1 à 6 atomes de carbone et
R⁶ signifie un hydrogène ou un radical alkyle avec 1 à 6 atomes de carbone,
en présence de 0,1 à 10 % en mole, rapportés à la quantité mise en oeuvre de composé de la formule A, d'un alcoolate alcalin ou alcalinoterreux avec 1 à 6 atomes de carbone.

2. Procédé selon la revendication 1, dans lequel l'isomérisation est effectuée en l'absence de diluant.

3. Procédé selon l'une quelconque des revendications précédentes, dans lequel l'isomérisation est effectué en présence de 0,5 à 8 % en mole, rapportés à la quantité mise en oeuvre de composé de la formule A, de l'alcoolate alcalin ou alcalinoterreux avec 1 à 6 atomes de carbone.

4. Procédé selon l'une quelconque des revendications précédentes, dans lequel l'alcoolate alcalin ou alcalinoterreux est un alcoolate de sodium ou de potassium avec 1 à 4 atomes de carbone.

5. Procédé selon l'une quelconque des revendications précédentes, dans lequel le composé de la formule A est un composé de la formule A1 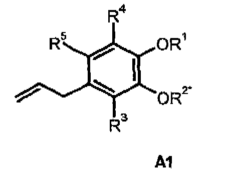 pour lequel on a :
R¹ signifie un radical alkyle avec 1 à 6 atomes de carbone ;
R^{2*} signifie un méthyle ;
R³, R⁴ et R⁵ signifient, respectivement indépendamment l'un de l'autre, un hydrogène ou un méthoxyle, avec la spécification qu'au moins un radical R³, R⁴ ou R⁵ signifie un hydrogène,
ou
R¹ et R^{2*} sont reliés ensemble, de telle sorte qu'un cycle est formé, de 5 ou 6 éléments en tout, qui comporte en tout 3 ou 4 atomes de carbone et 2 atomes d'oxygène,
R³, R⁴ et R⁵ signifient, respectivement indépendamment l'un de l'autre, un hydrogène ou un méthoxyle, avec la spécification qu'au moins un radical R³, R⁴ ou R⁵ signifie un hydrogène.

6. Procédé selon l'une quelconque des revendications précédentes, dans lequel le composé de la formule A est choisi parmi le groupe consistant en le naéthyl-eugénol l'éthyl-eugénol, l'iso-amylo-eugénol, l'élémicine, le safrole, la myristicine, l'apiol et le dillapiol.

7. Procédé selon l'une quelconque des revendications précédentes, dans lequel le rapport trans- : cis- des isomères à double liaison de la formule B est compris dans l'intervalle de 80 : 20 à 99 : 1, de préférence dans l'intervalle de 90 : 10 à 98 : 2.
